# DEMANDE DE BREVET EUROPEEN

(11) **EP 1 647 258 A1**
(43) Date de publication de la demande: **19.04.2006**
(21) Numéro de dépôt: 05292098.0
(22) Date de dépôt: 10.10.2005
(51) Int. Cl.: A61K 8/40, A61Q 5/06, A61K 8/19

(54) **Composition capillaire à base de monomères électrophiles et de micro- ou nanoparticule de lubrifiant solide**

(30) Priorité: 13.10.2004 FR 0411047
(71) Demandeur: L'OREAL, 75008 Paris (FR)
(72) Inventeur: Vic, Gabin, 60280 Venette (FR); Livoreil, Aude, 75006 Paris (FR)
(74) Mandataire: Bourdeau, Françoise

(57) **Abrégé**

La présente demande a pour objet une composition capillaire, caractérisée en ce qu'elle comprend, dans un milieu cosmétiquement acceptable, au moins un monomère électrophile et des micro- ou nanoparticules de lubrifiant solide.

## Description

La présente invention concerne de nouvelles compositions, destinées à conférer du volume aux cheveux, à base de monomères polymérisables in situ, d'un milieu cosmétiquement acceptable, et de micro- ou nanoparticules de lubrifiant solide, ainsi qu'à l'utilisation de ces compositions pour le traitement capillaire des matières kératiniques.

Par "matières kératiniques", on entend les cheveux.

Il existe de nombreux produits de coiffage permettant d'apporter du volume aux cheveux. Un inconvénient lié à ces produits, le plus souvent à base de polymères filmogènes, réside dans le fait que l'effet cosmétique disparaît dès le premier shampooing.

On connaît par ailleurs des traitements permanents des cheveux. Ces traitements utilisent un agent réducteur et un agent oxydant, et nécessitent une mise sous tension mécanique des cheveux à l'aide d'un matériel d'enroulage, afin de conférer une mise en forme

Ces procédés, qui permettent effectivement d'augmenter le volume de la chevelure, présentent toutefois l'inconvénient de modifier le niveau de frisure de la chevelure et de dégrader le toucher des cheveux, sans donner suffisamment de brillance et de lissage.

Il apparaît ainsi nécessaire de développer des compositions permettant d'accroître le volume de la chevelure sans modifier la forme ou le toucher des cheveux, le tout étant rémanent aux shampooings, et procurant aux cheveux de la brillance et du lissage.

La présente invention propose de nouvelles compositions, permettant de remédier à ces inconvénients.

La demanderesse a découvert, de manière surprenante, qu'en mettant en oeuvre des monomères électrophiles tels qu'ils sont décrits dans la demande de brevet FR2840208 et des micro- ou nanoparticules de lubrifiant solide, il était possible d'apporter du volume à la coiffure, de la brillance et du lissage des cheveux, sans dégradation des cheveux et sans adhésion des cheveux entre eux. En outre, ces propriétés cosmétiques sont rémanentes à plusieurs shampooings.

En plus du volume certaines particules permettent également d'apporter de manière rémanente à la chevelure de la brillance, du corps, de la masse, des effets optiques.

La demanderesse a également constaté qu'en appliquant une composition à base de tels monomères et de micro- ou nanoparticules de lubrifiant solide sur la chevelure, il se formait in situ un revêtement rémanent enrobant les objets.

L'invention a donc pour objet une composition capillaire, caractérisée en ce qu'elle comprend, dans un milieu cosmétiquement acceptable, au moins un monomère électrophile, et des micro- ou nanoparticules de lubrifiant solide.

Elle a également pour objet un procédé de traitement capillaire des cheveux, mettant en oeuvre cette composition.

Elle a également pour objet l'utilisation pour le traitement capillaire des cheveux, d'une composition comprenant, dans un milieu cosmétiquement acceptable, au moins un monomère électrophile, et des micro- ou nanoparticules de lubrifiant solide.

Elle a enfin pour objet un kit comprenant une première composition contenant au moins un monomère électrophile (présent à des teneurs pouvant être comprise entre 0,5 et 50% du poids de la première composition) et au moins un inhibiteur de polymérisation anionique et/ou radicalaire (présent à des teneurs pouvant être comprise entre 10 ppm et 5% du poids de la première composition), ainsi qu'une deuxième composition comprenant dans un milieu cosmétiquement acceptable des micro- ou nanoparticules de lubrifiant solide (présentes à des teneurs pouvant être comprise entre 0,001 et 4 % du poids de la deuxième composition).

D'autres objets de l'invention apparaîtront à la lecture de la description et des exemples qui suivent.

On entend par nanoparticule, toute particule dont la taille élémentaire est comprise entre 1nm et 999 nm et par microparticule toute particule dont la taille élémentaire est comprise entre 1µm et 300µm.

Cette nano- ou microparticule peut être sous forme d'une sphère, d'aiguilles, de flocons, de plaquettes, de tube, de fibre, de cube, de prisme ou avoir une forme irrégulière.

Par taille des particules, on entend la distance entre les deux points les plus éloignés de la particule.

Par « lubrifiant solide », on entend, au sens de la présente invention,tout matériau solide qui réduit la friction et/ou l'usure de deux surface en contact, en mouvement l'une par rapport à l'autre. De tels composés sont décrits dans l'encyclopédie Ullmann de Chimie Industrielle 2002, Section « Solide Lubricant », auteur Christian Busch ; et dans l'encyclopédie Kirk Otmer de Chimie Technologique, dans l'article « Lubrication and Lubricants », Section 3Solid Film Lubricants », auteur ER Boares.

On peut citer comme nano- ou microparticule à titre non limitatif les nanoparticules semiconductrices électroluminescentes (ou Quantum Dots), les nano ou microfibrilles, les microplaquettes, les latex, les nanotubes, les microobjets adhésifs, les particules expansibles.

Ces particules peuvent être minérales ou organiques.

De préférence, les nano- ou microparticule sont choisies parmi les particules de :
- Graphite, fluorure de graphite, KC8 (graphite de potassium), LiC8 (graphite de lithium)
- Bisufure de molydène (MoS2)
- Nitrure de bore
- Bisulfure d'étain (SnS2)
- BaSi2
- Silicate Si2O5
- Disulfure de titane (TiS2)
- Li2Si2O5
- Fluorure de cérium (CeF3)
- Kaolinite
- Talc
- Pyrophyllite (Al2Si4O10(OH)2)
- Mica
- Chlorure de Zirconium (ZrCl2)
- Muscovite
- Sulfate d'argent (Ag2SO4)
- Montmorillonite
- Bille de silicone
- Borax (Na2B4O7)
- Polytéréphtales
- Polymères perfluoroalcoxy (PFA)
- Copolymère Fluoroéthylène Propène (FEP)
- Nylon

Les micro- ou nanoparticules de lubrifiant solide peuvent être présentes dans la composition à des teneurs comprises entre 0,0001 et 40% en poids, de préférence entre 0,001% et 20%, et encore de préférence entre 0,01% et 10% en poids du poids total de la composition.

Encore plus préférentiellement, les particules de lubrifiant solide sont minérales.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

### MONOMERES

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Le ou les monomères électrophiles présents dans la composition de l'invention peuvent être choisis parmi
- les dérivés benzylidene malononitrile (A), le 2-(4-chloro-benzylidene)-malononitrile (A1) le 2-cyano-3-phényl acrylate d'éthyle (B) le, 2-cyano-3-(4-chloro-phényl) acrylate d'éthyle (B1) décrits dans Sayyah, *J. Polymer Research,* 2000, p97

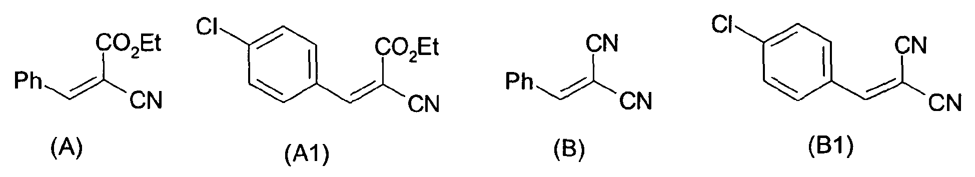

Les dérivés de méthylidenemalonates comme :
■ Le 2-méthylene-malonate de diéthyle (C) par Hopff, *Makromoleculare Chemie,* 1961, p95, De Keyser, J. *Pharm. Sci,* 1991, p67 et Klemarczyk, *Polymer,* 1998, p173 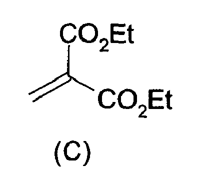
■ le 2-éthoxycarbonylméthyleneoxycarbonyl acrylate d'éthyle (D) par Breton, *Biomaterials,* 1998, p271 et Couvreur, *Pharmaceutical Research,* 1994, p1270.

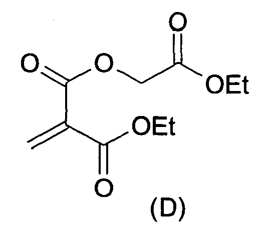

Les dérivés itaconate et itaconimide comme :
■ l'itaconate de diméthyle (E) par Bachrach, *European Polymer Journal,* 1976, p563 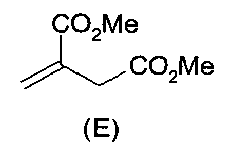
■ N-butyl itaconimide (F), N-(4-tolyl) itaconimide (G), N-(2-ethylphenyl) itaconimide (H) , N-(2,6-diethylphenyl) itaconimide (I) par Wanatabe, *J.Polymer Science : Part A :Polymer chemistry,* 1994, p2073 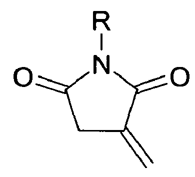 R= Bu (F), 4-tolyl (G), 2-ehylphenyl (H), 2,6-diethyphenyl (I)

Les dérivés α-(methylsulfonyl)acrylates de méthyle (K) , α-(methylsulfonyl)acrylates de d'éthyle (L) , α-(tert-butylsulfonyl)acrylates de méthyle (M) , α-(methylsulfonyl)acrylates de tert-butyle (N) α-(tert- butylsulfonyl)acrylates de tert-butyle (O) par Gipstein, *J.Org.Chem,* 1980, p1486 et les dérivés 1,1-bis-(methylsulfonyl)ethylene (P), 1-acetyl-1-methyl sulfonyl ethylene (Q) , α-(methylsulfonyl) vinyl sulfonate de methyle (R) , α-methylsulfonylacrylonitrile (S) par Shearer, US patent US2748050.

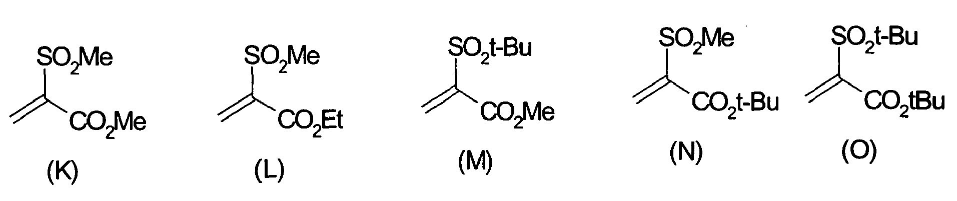

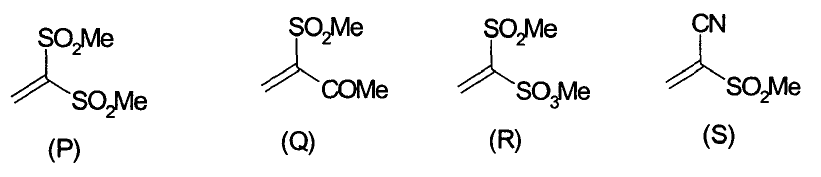

Les dérivés méthyl vinyl sulfone (T) et phényl vinyl sulfone (U) par Boor , *J. Polymer Science,* 1971, p249

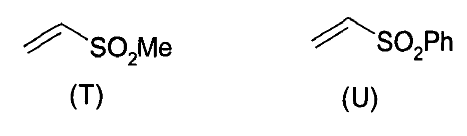

Le dérivé phényl vinyl sulfoxide (V) par Kanga, *Polymer preprints (ACS, Divison of Polymer Chemistry),* 1987, p322

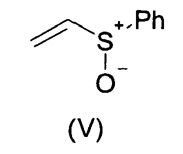

Le dérivé 3-methyl-N-(phenylsulfonyl)-1-aza-1,3-butadiene (W) par Bonner, *Polymer Bulletin,* 1992, p517

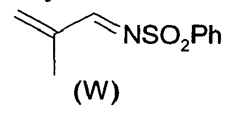

Les dérivés acrylates et acrylamides comme :
■ N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* 2005, p2754. 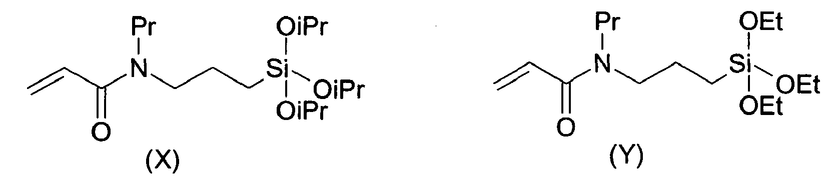
■ 2-hydroxyethyl acrylate (Z) et 2-hydroxyethyl méthacrylate (AA) par Rozenberg, *International Journal of Plastics Technology,* 2003, p17 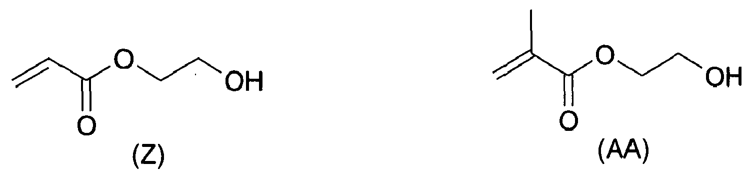
■ N-butyl acrylate (AB) par Schmitt, *Macromolecules,* 2001, p2115
■ Tert-butyl acrylate (AC) par Ishizone, *Macromolecules,* 1999, p955.

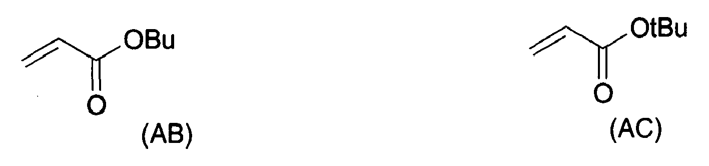

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (A) : 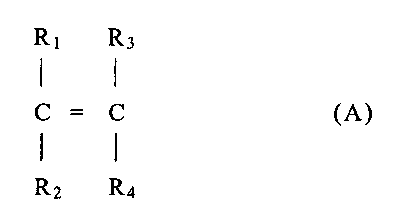 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
   R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
   R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère, R' désignant un radical alkyle en C₁-C₁₀, ce polymère pouvant être obtenu par polymérisation radicalaire, par polycondensation ou par ouverture de cycle.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)ₘ-CF₃ ou -(CH₂)n-(CF₂)m-CHF₂ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (B) : 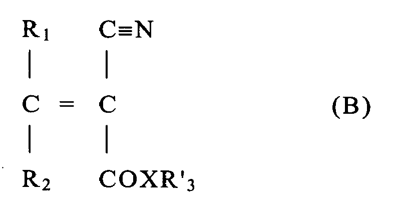
X désignant NH,S,O,
R₁ et R₂ ayant les mêmes significations que précédemment,
R'₃ pouvant désigner un atome d'hydrogène ou un radical R tel que défini pour la formule (A).
De préférence, X désigne O.

A titre de composés de formule (B), on peut citer les monomères :
a) appartenant à la famille des 2-cyanoacrylates de polyfluoroalkyle en C₁-C₂₀ tels que :
   l'ester 2,2,3,3-tétrafluoropropylique de l'acide 2-cyano-2-propénoïque de formule : 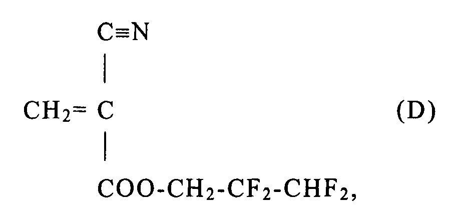
   ou encore l'ester 2,2,2-trifluoroéthylique de l'acide 2-cyano-2-propénoïque de formule : 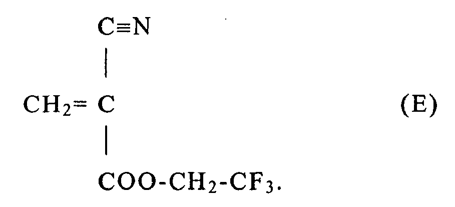
b) les cyanoacrylates d'alkyle en C₁-C₁₀ ou d'(alcoxy en C₁₋C₄)(alkyle en C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule F et leurs mélanges : 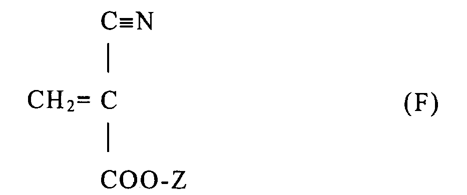 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Par milieu cosmétiquement acceptable, on entend un milieu compatible avec les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique, l'alcool béhénylique ; les acides gras en C₁₀-C₃₀ tels que l'acide laurique, l'acide stéarique ; les amides gras en C₁₀-C₃₀ tels que la diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 105 Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6 -trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, C10₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable.

Il est également possible, afin de moduler la cinétique de réaction, de préalablement humidifier les cheveux à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les cheveux à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des cheveux par transformation chimique de la matière kératinique.

A titre d'exemple, on peut citer la réduction des ponts disulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter les cheveux avec tous types de polymères, ou réaliser un traitement capillaire avant application de la composition de l'invention, comme une coloration directe ou d'oxydation, une permanente ou encore un défrisage.

L'application des compositions contenues dans le dispositif aérosol conforme à l'invention peut être suivie ou non d'un rinçage. Ces compositions peuvent se présenter sous des formes diverses, telles que de lotion, de spray, de mousse et être appliquées sous forme de shampooing ou d'après-shampooing.

Le mode d'application peut être en une seule étape ou bien être divisé en étapes successives. Si le procédé comporte plusieurs étapes d'application de compositions actives, ce peuvent être les suivantes :
1. Application sur les cheveux des micro- ou nanoparticules de lubrifiant solide, présentes en solution aqueuse à raison de 0,05 à 40 %, de préférence de 0.1 à 35 % et mieux de 0.25 à 25%
2. Application sur les cheveux humidifiés du monomère, présent en solution à une concentration comprise entre 0,05 et 30 % en poids, plus préférentiellement comprises entre 0,01 et 50 % en poids, et plus préférentiellement entre 0,1 et 20 % en poids.

En plus de l'actif, chaque composition peut contenir des additifs capillaires conventionnels. L'ordre des deux premières étapes peut être inversé. La première étape peut être précédée de l'application d'un produit capillaire : soin, shampooing, après shampooing, coloration, décoloration, permanente, défrisage. De même la dernière étape peut être succédée de l'application d'un produit capillaire. Chaque étape peut être interrompue par un rinçage, un séchage. Le séchage pouvant être effectué au casque, au sèche cheveux et/ou au fer à lisser.

L'invention concerne, en particulier, un procédé selon l'une quelconque des revendications précédentes, comprenant au moins les deux étapes :
- une première étape qui comprend l'application d'une composition contenant des micro- ou nanoparticules d'au moins un lubrifiant solide;
- une seconde étape qui comprend l'application d'une composition contenant au moins un monomère électrophile.

Avantageusement, la première et la seconde étape sont inversées.

L'invention a également pour objet l'utilisation des compositions décrites ci-dessus pour le traitement capillaire des matières kératiniques, et notamment des cheveux. Les compositions peuvent notamment être utilisées pour le renforcement des cheveux.

Les exemples qui suivent sont destinés à illustrer l'invention, sans toutefois présenter un caractère limitatif. Des essais ont été réalisés en utilisant le composé suivant : monomère de 2-cyanoacrylate de n-octyle, commercialisé sous la dénomination RITE LOK CON895 par la société CHEMENCE.

### Composition 1

| | |
|---|---|
| 2-Octyl cyanoacrylate | 10% m.a. |
| CYCLOPENTASILOXANE | 45% |
| Mélange CYCLOPENTASILOXANE DIMETHICONE COPOLYOL | 45% |

### Composition 2 de l'invention

| | |
|---|---|
| 2-Octyl cyanoacrylate | 10% m.a. |
| CYCLOPENTASILOXANE | 43% |
| CYCLOPENTASILOXANE DIMETHICONE COPOLYOL | 43% |
| Nitrure de bore | 4 % |

### Mode d'application

Des mèches constituées de 2,7 g de cheveux sensibilisés sont humidifiées à l'aide d' 1 ml d'eau par mèche. 2 g des compositions décrites ci-dessus sont appliqués sur ces mèches humidifiées. Après application les mèches de cheveux sont séchées au casque 30 minutes à 40°C.

Pour chaque mèche, le toucher et la brillance des cheveux sont évalués par un panel de 10 personnes. Une mèche vierge de même sensibilisation est utilisée comme référence. L'évaluation tactile et visuelle des diverses mèches de cheveux est répétée avec la même procédure après avoir réalisé successivement 5 shampooings commercialisés sous la dénomination DOP camomille.

| | | | |
|---|---|---|---|
| Evaluation sensorielle | Nature du Traitement | Composition 1 | Composition2 |
| | Après Application | Douceur 3 Démêlage 3 | Douceur 5 Démêlage 5 |
| | Après 5 shampooings | Douceur 2 Démêlage 2 | Douceur 4 Démêlage 4 |

| | | | |
|---|---|---|---|
| Notation : 0 = équivalent à la mèche non traitée 5 = très supérieur à la mèche non traitée | | | |

L'expérience montre que les modifications sensorielles (douceur et démêlage) apportées par la composition de l'invention sont supérieures à la composition 1 sans nitrure de bore juste après application des compositions. De plus après réalisation de 5 shampooings, la rémanence de l'apport de douceur et de l'apport de démêlage apportés par la composition de l'invention est conservée et supérieure à la composition sans nitrure de bore.

A noter que les niveaux de brillance et de démêlage sont plus élevés avec la composition de l'invention si celle-ci est appliquée sur cheveux mouillés.

### 1. Monomère methylheptylcyanoacrylate

| | |
|---|---|
| Methylheptylcyanoacrylate (1) | 10% m.a. |
| CYCLOPENTASILOXANE | 43% |
| CYCLOPENTASILOXANE DIMETHICONE COPOLYOL | 43% |
| Nitrure de bore | 4 % |

| | |
|---|---|
| (1) commercialisé par la société Chemence | |

### 2. Monomère methylheptylcyanoacrylate + acide acétique

| | |
|---|---|
| Methylheptylcyanoacrylate (1) | 10 % m.a. |
| Acide acétique | 0.25 % |
| CYCLOPENTASILOXANE | 42.75 % |
| CYCLOPENTASILOXANE DIMETHICONE COPOLYOL | 43 % |
| Nitrure de bore | 4 % |

| | |
|---|---|
| (1) commercialisé par la société Chemence | |

### 4. Monomère ethoxyethylcyanoacrylate

| | |
|---|---|
| ethoxyethylcyanoacrylate (1) | 10% m.a. |
| CYCLOPENTASILOXANE | 43% |
| CYCLOPENTASILOXANE DIMETHICONE COPOLYOL | 43% |
| Nitrure de bore | 4 % |

| | |
|---|---|
| (1) EO 460 commercialisé par la société Tong Shen | |

### 5. Monomère butylcyanoacrylate

| | |
|---|---|
| Butylcyanoacrylate (1) | 10% m.a. |
| CYCLOPENTASILOXANE | 43% |
| CYCLOPENTASILOXANE DIMETHICONE COPOLYOL | 43% |
| Nitrure de bore | 4 % |

| | |
|---|---|
| (1) B 60 commercialisé par la société Tong Shen | |

### 7. Monomère ethylhexylcyanoacrylate

| | |
|---|---|
| Ethylhexylcyanoacrylate (1) | 10% m.a. |
| CYCLOPENTASILOXANE | 43% |
| CYCLOPENTASILOXANE DIMETHICONE COPOLYOL | 43% |
| Nitrure de bore | 4 % |

| | |
|---|---|
| (1) O-60 commercialisé par la société Tong Shen | |

### 9. Mélange de monomère methylheptylcyanoacrylate et ethylhexylcyanoacrylate

| | |
|---|---|
| methylheptylcyanoacrylate (1) | 9 % m.a. |
| Ethylhexylcyanoacrylate (2) | 1 % |
| CYCLOPENTASILOXANE | 43 % |
| CYCLOPENTASILOXANE DIMETHICONE COPOLYOL | 43 % |
| Nitrure de bore | 4 % |

| | |
|---|---|
| (1) commercialisé par Chemence | |
| (2) O-60 commercialisé par la société Tong Shen | |

### 10. Mélange de monomère methylheptylcyanoacrylate et Butylcyanoacrylate

| | |
|---|---|
| methylheptylcyanoacrylate (1) | 7 % m.a. |
| butylcyanoacrylate (2) | 3 % |
| CYCLOPENTASILOXANE | 43% |
| CYCLOPENTASILOXANE DIMETHICONE COPOLYOL | 43% |
| Nitrure de bore | 4 % |

| | |
|---|---|
| (1) commercialisé par Chemence | |
| (2) B-60 commercialisé par la société Tong Shen | |

## Revendications

1. Composition capillaire, **caractérisée en ce qu'**elle comprend, dans un milieu cosmétiquement acceptable, au moins un monomère électrophile et des micro- ou nanoparticules de lubrifiant solide.

2. Composition selon la revendication 1, **caractérisée en ce que** le ou les monomères électrophiles répondent à la formule : 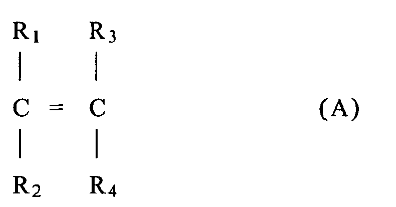 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, - S(R)₂⁺, -SH₂⁺, -NH₃⁺, -NO₂, -SO₂R, -C≡N, -COOH, -COOR, -COSR, - CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁- C₄, les groupements aryle et aryloxy,
R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R' désignant un radical alkyle en C₁-C₁₀.

3. Composition selon la revendication 2, **caractérisée en ce que** le ou les monomères électrophiles sont choisis parmi les composés de formule : 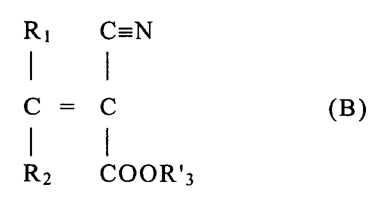
R₁ et R₂ sont tels que définis dans la revendication 3,
R'₃ désigne un atome d'hydrogène ou un radical R tel que défini dans la revendication 2.

4. Composition selon la revendication 3, **caractérisée en ce que** le ou les monomères sont choisis parmi les 2-cyanoacrylates de polyfluoroalkyle en C₁-C₂₀, les cyanoacrylates d'alkyle en (C₁-C₁₀) ou d'(alcoxy en C₁-C₄)(alkyle en C₁-C₁₀).

5. Composition selon la revendication 4, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

6. Composition selon la revendication 3, **caractérisée en ce que** le ou les monomères électrophiles répondent à la formule (F) : 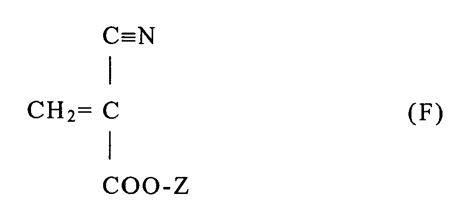 dans laquelle : Z=-(CH₂)₇-CH₃,
- CH(CH₃)-(CH₂)₅-CH₃,
- CH₂-CH(C₂H₅)-(CH₂)₃-CH₃,
- (CH₂)₅-CH(CH₃)-CH₃,
- (CH₂)₄-CH(C₂H₅)-CH₃.

7. Composition selon l'une quelconque des revendications précédentes, **caractérisée en ce que** le monomère est présent dans la composition à des teneurs comprises entre 0,001 et 80% en poids, de préférence entre 0,1 et 40%, et encore de préférence entre 1 et 20% en poids du poids total de la composition.

8. Composition selon l'une quelconque des revendications précédentes, **caractérisée en ce que** les monomères sont fixés de façon covalente sur des supports tels que des polymères, des oligomères ou des dendrimères.

9. Composition selon l'une quelconque des revendications précédentes, **caractérisée en ce que** le milieu cosmétiquement acceptable est anhydre.

10. Composition selon la revendication 9, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

11. Composition d'une composition selon l'une quelconque des revendications précédentes, **caractérisée en ce que** la composition comprend des inhibiteurs de polymérisation.

12. Composition selon la revendication 11, **caractérisée en ce que** les inhibiteurs sont des inhibiteurs de polymérisation anioniques et/ou radicalaires.

13. Composition selon la revendication 11, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

14. Composition selon l'une quelconque des revendications 11 à 13, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

15. Composition selon l'une quelconque des revendications précédentes, **caractérisée par le fait que** les particules sont minérales ou organiques.

16. Composition selon l'une quelconque des revendications précédentes, **caractérisée en ce que** les micro- ou nanoparticules sont choisies parmi les nano- ou microparticule sont choisies parmi les particules de :
- Graphite, fluorure de graphite, KC8 (graphite de potassium), LiC8 (graphite de lithium)
- Bisufure de molydène (MoS2)
- Nitrure de bore
- Bisulfure d'étain (SnS2)
- BaSi2
- Silicate Si205
- Disulfure de titane (TiS2)
- Li2Si2O5
- Fluorure de cérium (CeF3)
- Kaolinite
- Talc
- Pyrophyllite (A12Si4O10(OH)2)
- Mica
- Chlorure de Zirconium (ZrCl2)
- Muscovite
- Sulfate d'argent (Ag2SO4)
- Montmorillonite
- Bille de silicone
- Borax (Na2B4O7)
- Polytéréphtales
- Polymères perfluoroalcoxy (PFA)
- Copolymère Fluoroéthylène Propène (FEP)
- Nylon

17. Composition selon l'une quelconque des revendications précédentes, **caractérisée en ce que** les micro- ou nanoparticules sont présentes dans la composition à des teneurs comprises entre 0,0001 et 40% en poids, de préférence entre 0,001 et 20%, et encore de préférence entre 0,01 et 10% en poids du poids total de la composition.

18. Composition selon la revendication 17, **caractérisée en ce que** l'agent est encapsulé.

19. Composition selon l'une quelconque des revendications précédentes, **caractérisée en ce que** la composition est sous forme de lotion, de spray ou de mousse.

20. Utilisation d'une composition selon l'une quelconque des revendications précédentes pour le traitement cosmétique des cheveux.

21. Utilisation d'une composition selon l'une quelconque des revendications 1 à 19 pour le renforcement des cheveux.

22. Procédé de traitement des matières kératiniques, **caractérisé en ce qu'**on applique sur les matières kératiniques une composition selon l'une quelconque des revendications 1 à 19, en présence d'un agent nucléophile.

23. Procédé selon la revendication 22, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PHNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, CI⁻, RCOO⁻, SCN⁻, ROH, RSH, NCO⁻, CN⁻, NO₃⁻, ClO₄⁻ et H₂O, Ph représentant le groupe phényle, Ar représentant un groupe aryle et R représentant un groupe aryle en C₁-C₁₀.

24. Procédé selon la revendication 23, **caractérisé en ce que** l'agent nucléophile est l'eau.

25. Procédé selon l'une quelconque des revendications 22 à 25, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

26. Procédé selon l'une quelconque des revendications 22 à 25, **caractérisé en ce que** les matières kératiniques sont pré-imprégnées à l'aide d'un agent nucléophile.

27. Procédé selon l'une quelconque des revendications 22 à 26, **caractérisé en ce que** les matières kératiniques sont préalablement réduites avant application de la composition.

28. Procédé selon la revendication 27, **caractérisé en ce que** l'agent réducteur est choisi parmi le thiosulfate de sodium anhydre, le métabisulfite de sodium en poudre, la thiourée, le sulfite d'ammonium, l'acide thioglycolique, l'acide tiolactique, le thiolactate d'ammonium, le mono-thioglycolate de glycérol, le thioglycolate d'ammonium, le thioglycérol, l'acide 2,5-dihydroxybenzoique, le di-thioglycolate de diammonium, le thioglycolate de strontium, le thioglycolate de calcium, le formo-sulfoxylate de zinc, le thioglycolate d'isooctyle, la di-cystéine, le thioglycolate de monoéthanolamine.

29. Procédé selon l'une des revendications 23 à 28,
**caractérisé en ce que** la composition comprend en outre un polymère choisi parmi le poly(méthacrylate de méthyle) et les copolymères à base de cyanoacrylates.

30. Procédé selon l'une des revendications 23 à 29, **caractérisé en ce que** l'application de la composition est suivie d'un rinçage.

31. Procédé selon l'une quelconque des revendications précédentes, comprenant au moins les deux étapes :
- une première étape qui comprend l'application d'une composition contenant des micro- ou nanoparticules d'au moins un lubrifiant solide tel que défini dans l'une quelconque des revendications 1 et 15 à 16 ;
- une seconde étape qui comprend l'application d'une composition contenant au moins un monomère électrophile tel que défini à l'une quelconque des revendications 1 à 8.

32. Procédé selon la revendication 31, dans lequel la première et la seconde étape sont inversées.

33. Kit comprenant une première composition contenant au moins un monomère électrophile défini selon l'une quelconque des revendications 1 à 20 et au moins un inhibiteur de polymérisation anionique et/ou radicalaire, ainsi qu'une deuxième composition comprenant dans un milieu cosmétiquement acceptable des micro- ou nanoparticules de lubrifiant solide.
